# EUROPÄISCHE PATENTANMELDUNG

(11) **EP 2 022 398 A1**
(43) Veröffentlichungstag der Anmeldung: **11.02.2009**
(21) Anmeldenummer: 07747933.5
(22) Anmeldetag: 15.05.2007
(51) Int. Cl.: A61B 5/08, A63B 23/18

(54) **ATEMKONTROLLVERFAHREN ZUR EINWIRKUNG AUF EINEN ORGANISMUS**

(30) Priorität: 17.05.2006 RU 2006116846
(71) Anmelder: Shalygin, Vyacheslav Yrievich, Dedovsk, Moskovskaya obl. 143532 (RU)
(72) Erfinder: Shalygin, Vyacheslav Yrievich, Dedovsk, Moskovskaya obl. 143532 (RU)
(74) Vertreter: Jeck, Anton
(86) Internationale Anmeldenummer: PCT/RU2007/000237
(87) Internationale Veröffentlichungsnummer: WO 2007/133121

(57) **Zusammenfassung**

Die Erfindung bezieht sich auf ein Verfahren zur rhythmischen Atmung. Ist nach der Erfindung vorgesehen, dass die Phasendauer des Atmungszyklus nach einem Signal einer Mobileinrichtung bei unterschiedlichen Tätigkeiten (Funktionsaktivitäten) eingestellt wird, wobei die Phasendauer des Atmungszyklus innerhalb eines großen Zeitintervalls je nach dem Bestimmungszweck der Regelung geregelt wird, dann werden die Abstimmung des Atemrhythmus und der Herzkontraktionsfrequenz bei der Ausführung von Atmungsübungen bequem und einfach aufeinander abgestimmt.

## Beschreibung

Die Erfindung bezieht sich auf ein Verfahren zur rhythmischen Atmung.

Die Erfindung betrifft die alternative Vitalmedizin, welche die Atemübungen der Yogis benutzt. Sie kann im Rahmen eines sportlichen Trainingsablaufs als zusätzliche Übungsart des Kardio-Respirationssystems sowie als Mittel zur therapeutischen Einwirkung mittels Änderung des Atemrhythmus eingesetzt werden.

Es sind verschiedene Verfahren zur sportlichen und medizinischen Einwirkung auf den Körper mittels des Einflusses auf den Atmungsablauf bekannt. Sie können in drei Hauptgruppen eingeteilt werden.

Der Sauerstoffanteil in der Einatmungsluft kann beim Normaldruck der Einatmungsluft oder beim Unterdruck und dementsprechend mit geringerer Sauerstoffaufnahme abgebaut werden.

Um diese Verfahren anzuwenden, werden Druckkammern (Erzeugung des Unterdrucks) und verschiedene Einrichtungen eingesetzt. Diese Einrichtungen stellen einen reduzierten Sauerstoffgehalt in der Einatmungsluft sicher. Die Konstruktion dieser Einrichtungen enthält solche Elemente, welche die Atmungsorgane und die atmosphärische Luft voneinander trennen. (Chizhov A.Ya., Karash Yu.M. Verfahren zur Erhöhung der unspezifischen Widerstandsfähigkeit des Körpers. Urheberschein zur Erfindung Nr. 1264949. M., 1986. Erfindungsblatt 1986. 39 und Chizhov A.Ya., Karash Yu.M., Filimonov V.G., Strelkov R.B. Verfahren zur Erhöhung der kompensatorischen Körpermöglichkeiten. Urheberschein zur Erfindung Nr. 950406. Erfindungsblatt, 1982. 30. S. 33-34). Die Hypoxieerzeugung mittels verschiedener Atmungstechniken, z. B. einer Yogisübung Pranayama und das Verfahren zur willensmäßigen Atmungsregelung für Bronchialasthmakranke (K.P. Buteyko) u. a. m. sind bekannt.

Die Hypoxieerzeugung mittels des rhythmischen Atmens sowie die Rhythmussteuerung werden durch verschiedene Geräte vorgenommen. (Z. B. "Verfahren zur willkürlichen Atmungsregelung bei Bronchialasthmakranken" RU 2141251 C1 und "Verfahren zur Behandlung von Hypoxie - Die diskontinuierliche Resonanzbehandlung von Hypoxie" RU 2197280 C2 u. a. m).

Jedes dieser Verfahren weist seine Vor- und Nachteile auf. Jedoch lassen sich folgende gemeinsame Mängel erkennen: Es ist erforderlich, gewisse Zeit speziell für die Durchführung der Behandlungen unter Einsatz von Sondergerätetechnik (Druckkammern, Computer-Trainingsanlagen u. a. m.) sowie für den Besuch von Heilanstalten einzuplanen. Die Kompliziertheit bei der Einweisung in die gerätelose Atmungsregelung sowie der große Zeitaufwand im Zusammenhang mit der Schulung und Durchführung des Unterrichts ist ein weiterer Mangel. Das begrenzt die Zeit, wo der Körper des Benutzers im Hypoxie-Zustand verweilen kann. Das ändert auch die etablierte Lebensweise des Benutzers, weil er anstatt seines konventionellen Tagesablaufs gewisse Zeit für solche Heil- und Übungsmaßnahmen in Anspruch nehmen muss.

Es ist Aufgabe der Erfindung ein Verfahren zu schaffen, das diese Mängel beseitigt. Gemäß diesem Verfahren können dann die Trainings- und Heilaktivitäten zu jeder Zeit und gleichzeitig mit beliebigen Tätigkeiten und zwar langzeitig durchgeführt werden (z. B. beim Lesen, während der Arbeit auf dem PC, beim Spazierengehen usw.). Dies erfolgt, ohne den gewöhnlichen Lebensrhythmus des Benutzers ändern zu müssen.

Das einfache Verfahren zur Atemrhythmusregelung: Die Aneignung dieses Verfahrens braucht ungefähr solange wie das Beibringen der Bedienung der Bedienungseinheit bei der Haushaltstechnik. Dieses Verfahren funktioniert nach dem "Autopilot"-Prinzip. Es setzt praktisch keine Konzentration der Aufmerksamkeit voraus. Es wird als eine bedingt-reflektorische Reaktion ausgeführt. Der Atmungszyklus (beliebige Phase des Atmungszyklus) erfolgt mit einem Steuersignal. Die Anwendung dieses Verfahrens ist für andere Personen unauffällig.

Der nächste Stand der Technik ist ein Verfahren des rhythmischen Atmens der Yogis. Dieses Verfahren wurde als Prototyp gewählt. Bei diesem Verfahren wird eine beliebige Phase des Atmungszyklus - Einatmen, Ausatmen, Atemanhalten - innerhalb einer bestimmten Anzahl von Herzkontraktionen ausgeführt.

Der Mangel dieses Verfahrens ist die Notwendigkeit, immer und unterbrochen auf das Zählen der Herzkontraktionen und auf das Einprägen des Herzrhythmus im Gedächtnis aufzupassen.

Der rhythmische Atem der Yogis erfolgt im statischen unbeweglichen Zustand. Dabei ist der Einfluss der Außen- und der inneren Faktoren praktisch ausgeschlossen.

Deswegen ist die Frequenzänderung der Herzkontraktionen (HKF) sehr gering und beträgt ca. 3 - 10 %. Bei der Änderung des Funktionszustands (z. B. infolge der Bewegungen und/oder infolge der nervalen Beanspruchungen) können die Abweichungen der Herzkontraktionsfrequenz (HKF) sogar 300 % (von statischen Ausgangswert) erreichen (d. h., es liegt ein weiter Änderungsbereich vor). Darum ist es unmöglich, bei solchem Atmen den Sauerstoffmangel im Körper infolge des sich abwechselnden Rhythmus - d. h., der Herzkontraktionsfrequenz zu erzeugen (es ist schwer, diesen Rhythmus zu ermitteln). Dabei wird die ganze Aufmerksamkeit nur auf die Abstimmung des Atemrhythmus mit der Herzkontraktionsfrequenz fokussiert sein. In einem solchen Atemzustand sind keine Aktivitäten möglich.

Die Schwierigkeiten beim Beibringen des Verfahrens sowie die Probleme bei der Konzentration der Aufmerksamkeit innerhalb von 2 bis 20 Minuten stellen ein empfindliches Hindernis für viele Benutzer dar, die das rhythmische Atmen anwenden möchten. Deswegen nimmt die Aneignung dieses Verfahrens einige Monate oder sogar Jahre in Anspruch. Die Zeit, wo der Organismus sich im Hypoxie-Zustand befindet, ist sehr begrenzt. Es gibt auch viel zu wenig Leute, die wegen Zeitmangels und der Kompliziertheit des rhythmischen Atmens dieses Verfahren langfristig im Laufe des Tages ausführen werden.

Das Ziel der Erfindung ist die Entwicklung eines Verfahrens zur Einwirkung auf den Körper. Die Einwirkung sollte mittels der Änderung der Menge der beim Atmen eintretenden Luft innerhalb eines breiten Bereichs und mittels der Ausführung von einfacheren und bequemeren Atemübungen der Yogis (Pranayama) erfolgen. Diese Übungen ändern den Atemrhythmus ab. D. h., sie verlängern bzw. verkürzen einzelne oder mehrere Phasen des Atmungszyklus. Somit üben sie die Trainings- und die Heilwirkung auf den Körper aus. Das weitere Ziel ist die Erhöhung der Wirksamkeit und die Minimierung des Zeitaufwands. Bei der Anwendung dieser Übungen, um die Gesundheit aufrechtzuerhalten und die Sportler zusätzlich zu trainieren, wird die natürliche Lebensweise gestört.

Die technischen Ergebnisse der Erfindung sind wie folgt:
1. Bequemes und einfaches Verfahren der Abstimmung des Atemrhythmus und der Herzkontraktionsfrequenz zur Ausführung der Atmungsübungen auf der Basis des Yogi-Systems,
2. Die Bildung und die Aufrechterhaltung von dosierter Hypoxie im Körper des Benutzers innerhalb einer langen Zeit,
3. Die Verlängerung der Verweilzeit in diesem Zustand der dosierten Hypoxie, ohne die natürliche Lebensweise zu stören,
4. Objektivität bei der Verfolgung der Sauerstoffbedarfsänderungen im Körper je nach dem Einfluss der sich abwechselnden Außen- und inneren Faktoren,
5. Die Erhöhung der Einwirkungsstufe auf den Körper,
6. Die Gebrauchseigenschaften im Zusammenhang mit der Verfahrensanwendung bei verschiedenen Tätigkeiten. Das betrifft vor allem die Unauffälligkeit für andere Menschen bei der Anwendung des Verfahrens und
7. Die Möglichkeit, das Verfahren zur Durchführung der Hyperventilation einzusetzen.

Viele Atemübungen der Yogis beruhen auf der Abstimmung des Atemrhythmus und der Herzkontraktionsfrequenz. Das Wesen dieser Abstimmung besteht in der Ausführung von einer oder mehreren Phasen des Atmungszyklus innerhalb einer bestimmten Anzahl von Herzkontraktionen. Unter den Phasen des Atmungszyklus werden Einatmen, Ausatmen, Atemanhalten nach dem Einatmen oder dem Atemanhalten nach dem Ausatmen verstanden. Bei verschiedenen Modifikationen des rhythmischen Atmens kann das Atemanhalten einmal oder mehrmals ausgelassen werden.

Im Allgemeinen wird aber die Anwendung des Verfahrens bei allen Phasen des Atmungszyklus betrachtet. Neben dem Zählen der Anzahl von Herzkontraktionen während der Ausführung der Atemübungen der Yogis muss noch auf die Richtigkeit des Ablaufs des eigentlichen Atemvorgangs sowie auf die Energiesteuerung (Prana) genau aufgepasst werden. Die Prana-Energie tritt mit der eingeatmeten Luft ein. Eine solche Verteilung der Aufmerksamkeit erschwert für einen modernen Menschen die Aneignung und die Anwendung der Atemübungen der Yogis. Die Lehrzeit (die Anzahl von Trainings) im Zusammenhang mit der Richtigkeit der Ausführung der Übungen wird verlängert. Vielen Leuten mangelt es an Geduld, und sie hören den Unterricht auf. Es ist viel bequemer, die mechanische Arbeit im Zusammenhang mit dem Zählen der Anzahl der Herzkontraktionen der Gerätetechnik (d. h., den Einrichtungen) zuzuweisen. Dabei muss man sich mehr auf die Richtigkeit des eigentlichen Atmungsvorgangs und die Prana-Steuerung konzentrieren. D. h., die Einrichtung muss die Herzkontraktionen erfassen, die erforderliche Anzahl der Herzkontraktionen auszählen und das Steuersignal (z. B. am Anfang der Ausführung der nächsten Phase des Atmungszyklus) ausgeben. Demgemäß wird der Zeitabstand zwischen dem Anfang der einen Phase bis zum Anfang der nächsten Phase der erforderlichen Anzahl der Herzkontraktionen entsprechen. Diese erforderliche Anzahl der Herzkontraktionen für die Ausführung einer oder mehrerer Phasen des Atmungszyklus wird in der Einrichtung eingestellt (sie wird für eine bestimmte Zeitdauer je nach dem jeweiligen Ziel vorgegeben z. B. für ein Training, für einen Tag, für eine Woche usw.).

Die Ausführung der Atemübungen wird dann bequemer und einfacher. Die Phasen des Atmungszyklus werden nach dem Außensteuersignal ausgeführt. Somit ist es nicht mehr erforderlich, an das Auszählen der Herzkontraktionen zu denken. Als Ergebnis der Verfahrensanwendung wird praktisch die ganze Aufmerksamkeit auf die Richtigkeit der Ausführung der Übungen (z. B. die Reihenfolge der Ausfüllung von verschiedenen Lungenbereichen mit Einatmungsluft) und auf die Energiesteuerung gerichtet.

Der rhythmische Atem der Yogis und die verschiedenen Modifikationen dieser Übung, bis auf die Abstimmung des Atemrhythmus und des Herzrhythmus, besteht in der allmählichen Verlängerung der Phasendauer des Atmungszyklus je nach der Geübtheit (d. h., die Phasen des Atmungszyklus werden während der größeren Anzahl der Herzkontraktionen ausgeführt). Die Phasenverlängerung des Atmungszyklus beim rhythmischen Atmen erzeugt im Vergleich zum Normalatmen einen gewissen (dosierten) Sauerstoffmangel im Körper. Um die Phasendauer des Atmungszyklus nach diesem Verfahren zu verlängern, muss in der Einrichtung eine größere Anzahl von Herzkontraktionen vorgegeben werden, im Verlauf derer einzelne Phasen des Atmungszyklus ausgeführt werden müssen.

Der Sauerstoffmangel (Hypoxie) und die Hyperventilation (übermäßige Sauerstoffaufnahme beim Atmen) werden weit und breit in sportlichen und medizinischen Anwendungen benutzt.

Der moderne Mensch ist nicht immer (und sogar ziemlich selten) imstande, aufgrund seines etablierten Lebensrhythmus, die Zeit zum Lernen und zur andauernden Durchführung der Atemübungen der Yogis zu finden und somit den positiven Effekt für seine Gesundheit zu bekommen. Der Besuch von Heil- und Genesungsanstalten zur Hypoxiebehandlung erfordert viel Zeitaufwand und stört den gewöhnlichen Lebensrhythmus.

Deswegen ermöglicht das vorgeschlagene Verfahren, einen vorgegebenen (dosierten) Sauerstoffmangel im Körper zu erzeugen und aufrechtzuerhalten. Dabei können gleichzeitig andere Aktivitäten durchgeführt werden. Man braucht nicht mehr, die bestehende Lebensweise zu ändern und eine extra Zeit für die Übungen auf der Basis des Yogi-Systems in Anspruch zu nehmen. Dabei kann der positive Effekt von diesen Übungen erreicht werden, wenn gleichzeitig andere Aktivitäten vorgenommen werden. Durch die Überlappung (Gleichzeitigkeit) verschiedener Tätigkeiten und der Atmung mit regelbaren Phasenzeiten des Atmungszyklus wird die Dauer der Einwirkung des Sauerstoffmangels oder -Überschusses auf den Körper (je nach dem Ziel der Übungen) sichergestellt. Somit kann die Effizienz des Trainings- und/oder des Heil- und Genesungsvorgangs erhöht werden.

Diese Atmungsart kann zur Erzeugung von einem bestimmten (dosierten) Sauerstoffdefizit beim Spazierengehen, bei den Fahrten mit öffentlichen Verkehrsmitteln, beim Fernsehen und Lesen usw. eingesetzt werden.

Von diesem Standpunkt aus ist die Zweckbestimmung des Verfahrens die Modifikation der Benutzung des rhythmischen Atmens. Sie beruht auf der Regelung der Phasenzeiten des Atmungszyklus. Diese Variante dient zur Erzeugung und langfristigen Aufrechterhaltung von einem bestimmten dosierten Sauerstoffmangel (oder Sauerstoffüberschuss, je nach dem Übungsziel) im Körper. Es kann gesagt werden, dass die Anwendung des Verfahrens bei der Ausführung des rhythmischen Atmens der Yogis die Regelung der Phasenzeiten des Atmungszyklus bei einer anderen Aktivität darstellt. Es handelt sich dabei um die Kontrolle der Richtigkeit der Atmung und die Energiesteuerung.

Gemäß diesem Bestimmungszweck des Verfahrens bei verschiedenen Tätigkeiten werden die Phase oder die Phasen des Atmungszyklus zeitlich (unter Abnahme der Atemfrequenz) im Vergleich zur Normalatmung verlängert. D. h., die Phasendauer des Atmungszyklus nach diesem Verfahren stellt den regelbaren Parameter in Bezug auf die aktuelle Tätigkeitsart (Funktionsaktivitäten) dar.

Die Realisierung des Verfahrens zur Atemregelung bei verschiedenen Tätigkeiten wird durch folgende Bedingungen sichergestellt.
1. Das Außensteuersignal dient zur Ausführung der Phase(n) des Atmungszyklus. Dieses Signal wird in einem Mobilgerät erzeugt. Dieses Gerät kann im aktiven Betriebszustand mitgetragen (oder in der Nähe des Benutzers angeordnet) werden, wie es bei Handys, Taschenempfängern, mobilen Audioplayern usw. der Fall ist. D. h., die Regelungsfunktion wird beansprucht, wenn die Einrichtung den Benutzer bei verschiedenen Tätigkeiten (Funktionsaktivitäten) begleitet. In diesem Sinn wird der Begriff "Mobileinrichtung" benutzt.
2. Um den bestimmten dosierten Sauerstoffmangel im Körper des Benutzers bei verschiedenen Tätigkeiten (Funktionsaktivitäten) zu erzeugen und aufrechtzuerhalten, wird die Phasendauer des Atmungszyklus gemäß der aktuellen funktionellen Aktivität im Vergleich zum gewöhnlichen Atmen bei der gleichen Funktionsaktivität (Tätigkeit) verlängert.
3. Die Ausführung einer oder mehrerer Phasen des Atmungszyklus erfolgt als eine bedingt-reflektorische Reaktion - die Ausführung der Phase(n) des Atmungszyklus als Erwiderung auf das Außensteuersignal. Nach einer gewissen Gewöhnungszeit wird ein Reflex eingeschliffen, gemäß einem Außensteuersignal (es dient als Bedingung) zu atmen. Ein solcher Regelablauf erfordert keine Aufmerksamkeit in Bezug auf den Atmungsvorgang. Deswegen kann diese Atmungsart mit zeitlich längerer Phase bzw. Phasen des Atmungszyklus bei gleichzeitiger Durchführung von anderen unterschiedlichen Tätigkeitsarten (Funktionsaktivitäten) angewendet werden.
4. Die Änderungen des Funktionszustandes des Organismus bei der Änderung der Funktionsaktivitäten (Tätigkeitswechsel) werden objektiv (ohne Eingriff des Benutzers) verfolgt. Verschiedene Tätigkeitsarten (Funktionsaktivitäten) bedürfen verschiedener Sauerstoffmengen für den Organismus des Benutzers. Mit anderen Worten, die erforderliche Menge des Sauerstoffs bei unterschiedlichen Funktionsaktivitäten hängt von Außen- und Innenfaktoren ab. Deswegen muss der Sauerstoffbedarf für die aktuellen Funktionsaktivitäten (Tätigkeitsart) berücksichtigt werden. Gemäß diesem Bedürfnis muss auch die jeweilige Hypoxie erzeugt und erhalten werden. Die HKF-Änderungen repräsentieren sachlich die Auswirkungen der Innen- und Außenfaktoren auf den menschlichen Organismus, darunter auch den Sauerstoffbedarf des Organismus. Deswegen ermöglicht die ununterbrochene Erfassung der HKF mittels eines Impulsgebers, die Änderungen der Funktionsaktivitäten und somit die Sauerstoffbedarfsänderungen im Körper je nach dem Einfluss der sich abwechselnden Außen- und inneren Faktoren zu verfolgen.
5. Dem Funktionsprinzip der Einrichtung liegt der Ablauf der Signalerzeugung je nach den jeweiligen Parametern des Organismus und den Kennwerten, die den Trainingsvorgang (die Einwirkung) festlegen, zugrunde. Ist die in der Mobileinrichtung vorgegebene Anzahl der auszuzählenden Herzkontraktionen geringer als beim normalen Atmen (d. h., die Atemfrequenz wird höher als beim Normalatmen sein), so wird der Körper eine überschüssige Sauerstoffmenge bekommen. Es handelt sich also um die Hyperventilation. Bei einigen Trainingsarten wird auch der Hyperventilationsbetrieb angewendet.

Indem in der Mobileinrichtung verschiedene Werte für die Anzahl der Herzkontraktionen vorgegeben werden, innerhalb deren die Phasen des Atmungszyklus ausgeführt werden müssen, wird ein breiter Regelungsbereich für die Phasenzeiten und die Erhöhung der Einwirkungsstufe auf den Körper sichergestellt (und zwar von der Hyperventilation bis zur Hypoxie verschiedener Größen).

Um dieses Verfahren zu realisieren, kann die Einrichtung zur Atemübung gemäß Patent RU 2183130 C2 («Elektronische Einrichtung zur Atemübung») benutzt werden.

### Diese Einrichtung umfasst folgende Funktionsbaugruppen:

Pulsgeber, Trainingsbelastungssteller, Herzkontraktionszähler und Anzeigeeinheit. Als Trainingsbelastung gilt das Verhältnis zwischen der Phase des Atmungszyklus und der Anzahl der Herzkontraktionen (diese Funktionsbaugruppe enthält auch eine Umschaltereinheit und einen Entschlüsseler). Diese Einrichtung hat geringe Abmessungen und ein geringes Gewicht. Deswegen kann diese Einrichtung praktisch unter beliebigen Bedingungen eingesetzt, mitgetragen bzw. in der Nähe gehalten werden.

Der Benutzer gibt im Trainingsbelastungssteller je nach dem Trainingsziel und nach seiner eigenen Geübtheit die Anzahl der Herzkontraktionen N vor. Während dieser Kontraktionen wird jede Phase des Atmungszyklus ausgeführt. D. h., die Dauer jeder Phase des Atmungszyklus wird mit der Zeit der N Kontraktionen des Herzens des Benutzers zusammenfallen. Das gleiche kann auch mit anderen Worten beschrieben werden und zwar: Die Belastung wird als Verhältnis der Phase(n) des Atmungszyklus zur Anzahl der Herzkontraktionen N vorgegeben.

### Der Algorithmus der Funktion der Einrichtung ist wie folgt:

Der Pulsgeber schickt die Impulse in die Einrichtung, direkt zum Herzkontraktionszähler. Erreicht die vorgegebene Summe im Zähler den N-Wert (welcher im Trainingsbelastungssteller gespeichert ist), so gibt die Einrichtung das Signal für den Anfang der nächsten Phase des Atmungszyklus aus. Danach wird der Zähler auf Null gesetzt. Sobald der erste Impuls (nach dem Rücksetzen des Zählers) vom Pulsgeber eintritt, zählt der Zähler wieder die Impulse aus, bis die Summe = N erreicht wird. Danach wird der Zähler wieder rückgesetzt und der ganze Vorgang wiederholt sich, solange die Einrichtung betrieben wird. Somit wird der Abstand zwischen den zwei aufeinander folgenden Signalen für den Anfang der nächsten Phasen des Atmungszyklus sichergestellt. Dieser Abstand fällt mit der Zeit zusammen, innerhalb der das Herz N Male kontraktiert. Nach dem Signal von der Mobileinrichtung beginnt der Benutzer, die jeweilige Phase des Atmungszyklus zeitgerecht auszuführen. Diese Zeit fällt mit der Auszählzeit N für die Herzkontraktionen in dieser Phase zusammen. Das nächste Signal meldet dem Benutzer den Anfang der nächsten Phase. Somit braucht man nur, die Signale von der Einrichtung zu befolgen (die jeweiligen Phasen auszuführen), ohne sich mit dem Auszählen der Herzkontraktionen zu bemühen. Die Abstimmung des Atemrhythmus und des Herzrhythmus setzt keine Anstrengungen voraus. Dadurch wird es bequemer und einfacher, die Übungen auszuführen.

Im Ruhezustand ist der Einfluss der Außen- und/oder der inneren Faktoren gering. Die Atemfrequenz beträgt 12 - 20 Atemzyklen und die HKF beträgt 60 - 80 pro Minute. Der gewöhnliche (natürliche) Atem weist zwei Phasen des Atmungszyklus auf, das Einatmen und das Ausatmen. Im Ruhezustand betragen diese Phasen ihrer Dauer nach ca. 1,5 - 2,5 Sek. D. h., während des Einatmens oder Ausatmens erfolgen ca. 2 - 3 Herzkontraktionen.

Wird die Phasendauer des Atmungszyklus vergrößert, zum Beispiel, wenn jede Phase (Einatmen und Ausatmen für die Zweiphasenatmung) 10 Sek. lang ist, entsteht im Körper ein bestimmtes Niveau von Sauerstoffmangel. Wenn die Phasendauer 10 Sekunden beträgt, wird die Atemfrequenz 3 Atemzyklen pro Minute betragen, und das Herz wird ca. 10 - 13mal pro Phase kontraktieren. Somit legt die Änderung der Anzahl der Herzkontraktionen den Zeitabstand zwischen zwei Signalen aus der Einrichtung fest (indem der N-Wert im Steller der Einrichtung höher oder weniger als beim Normaldruck vorgegeben wird). Da die Phasendauer von den Signalabständen abhängt, so werden auch der erforderliche Grad und die erforderliche Art der Einwirkung durch die Signalabstände festgelegt. Je seltener der Atem, desto ausgeprägter die Hypoxie. Der N-Wert kann je nach dem erforderlichen Einwirkungsgrad vorgegeben werden, zum Beispiel, im Bereich von 1 bis 600. Dadurch wird auch der große Zeitbereich für die Regelung der Phasenzeiten und des Einwirkungsgrades im Zusammenhang mit dem angewendeten Verfahren auf den Körper sichergestellt.

Je höher die Atemfrequenz, desto mehr Sauerstoff tritt mit der Luft in den Organismus ein. Um die Atmungsübungen der Yogis, zum Beispiel des rhythmischen Atmens, auszuführen, gibt der Benutzer die Trainingsbelastung in der Mobileinrichtung vor. D. h., der Benutzer gibt im Trainingsbelastungssteller den N Wert ein. Danach beginnt der Benutzer, die Übung innerhalb einer bestimmten Zeit auszuführen. Diese ganze Zeit lang wird die Einrichtung die Signale in bestimmten Abständen ausgeben. Die Abstände werden den N Herzkontraktionen gleich sein. Diese Signale informieren den Benutzer über die Notwendigkeit, die neue Phase des Atmungszyklus einzuleiten. Mit anderen Worten: Wenn das Signal von der Einrichtung eintrifft, fängt der Benutzer an, die Luft einzuatmen. Das Einatmen dauert solange, bis das nächste

Signal eintrifft. Nach diesem Signal beginnt das Ausatmen, das bis zum nächsten Signal dauert. Somit braucht der Benutzer nicht die Herzkontraktionen während der ganzen Übung auszuzählen. Die Einrichtung macht diese Arbeit von selbst. Die Abstimmung des Herzrhythmus und des Atemrhythmus wird vereinfacht. Deswegen kann dem eigentlichen Atemvorgang mehr Aufmerksamkeit geschenkt werden. Die Übungen können viel einfacher und bequemer ausgeführt werden.

Je nach dem Bedarf und der Geübtheit werden die Yogi-Atemübungen mit größerer Belastung ausgeführt. D. h., in der Einrichtung wird das größere Verhältnis für die Phase(n) des Atmungszyklus zur Anzahl der Herzkontraktionen (N-Wert) vorgegeben. Die gleiche Einrichtung kann auch im Verfahren zur Erzeugung von einer bestimmten dosierten Hypoxie in sportlichen und medizinischen Anwendungen eingesetzt sowie bei verschiedenen Tätigkeiten (Funktionsaktivitäten) verwendet werden. Im Zusammenhang mit der praktischen Anwendung können verschiedene Tätigkeitsarten (Funktionsaktivitäten) als eine Gesamtheit von statischen Zuständen (Aktivitäten) des Organismus betrachtet werden. Diese Aktivitäten werden für jeden Zustand durch seine eigenen HKF-Werte und Atemfrequenzen bei natürlicher Atmung gekennzeichnet (d. h., die Phasendauer kann der Anzahl der Herzkontraktionen für jeden Zustand gleichgesetzt werden). Wird der N-Wert in der Mobileinrichtung größer als die höchste Anzahl der Herzkontraktionen bei natürlicher Atmung aus allen möglichen statischen Zuständen vorgegeben, so kann die bestimmte Hypoxiestufe für die ganze Gesamtheit der statischen Zustände (Aktivitäten) des Organismus, d. h. aller möglichen Tätigkeitsarten erzeugt werden. Somit werden die Phasendauerregelung und die Einwirkung auf den Organismus für die ganze Gesamtheit der statischen Zustände (Aktivitäten) des Organismus realisiert. Da der Benutzer die Einrichtung nach diesem Verfahren ständig dabei hat, und die Impulse des Pulsgebers ständig in der Einrichtung ankommen, so wird die sachliche Verfolgung der HKF-Änderung (infolge der Änderung von Tätigkeitsart, Aktivität, der Einwirkung von Innen- und/oder Außenfaktoren) sichergestellt. Es wird auch die Phasenregelung und die Einwirkung auf den Organismus beim Übergang von einem statischen Zustand zum anderen innerhalb der ganzen Gesamtheit sichergestellt. Diese Änderungen der HKF verursachen die Änderung der Abstände in der Signalfolge von der Einrichtung. Die HKF-Änderungen repräsentieren die Reaktion des Organismus auf die neuen Sauerstoffbedürfnisse beim Wechsel der Aktivitäten (Tätigkeit). Somit wird die Anwendung des Verfahrens beim Übergang von einer Funktionsaktivität (Tätigkeitsart) zur anderen sichergestellt.

### Beispiel:

Wenn das Verfahren nicht eingesetzt wird (natürliche Atmung), werden für drei Funktionsaktivitäten folgende Nennwerte angenommen:
1. bei HKF = 60 Schläge/Min beträgt die Atemfrequenz (AF) 15 pro Minute; die Phasendauer beträgt 2 Sek., d. h., jede Phase dauert solange wie 2 Herzkontraktionen,
2. bei HKF = 90 Schläge/Min, beträgt die Atemfrequenz 23 pro Minute, die Phasendauer beträgt 1,3 Sek., d. h., jede Phase dauert ca. 2 Herzkontraktionen,
3. bei HKF = 110 Schläge/Min, beträgt die Atemfrequenz 28 pro Minute, die Phasendauer beträgt 1,1 Sek., d. h., jede Phase dauert ca. 2 Herzkontraktionen.

Im Trainingsbelastungssteller der Einrichtung wurde der N-Wert gleich 5 eingestellt (jede Phase wird innerhalb von 5 Herzkontraktionen ausgeführt).

Dementsprechend ist die Atemfrequenz beim Einsatz des Verfahrens für die drei Funktionsaktivitäten und bei der Zweiphasenatmung wie folgt:
1. AF = 6 pro Minute bei HKF = 60 Schläge/Min, die Phasendauer wird 5 Sek. betragen,
2. AF = 9 pro Minute bei HKF = 90 Schläge/Min, die Phasendauer wird 3,3 Sek. betragen,
3. AF = 11 pro Minute bei HKF = 110 Schläge/Min, die Phasendauer wird 2,7 Sek. betragen,

D. h., die Signale von der Mobileinrichtung für die drei Zustände werden in Abständen von 5 Sek., 3,3 Sek., 2,7 Sek. erzeugt, und die Phasendauer der Atemzyklen für diese drei Zustände wird jeweils 5 Sek., 3,3 Sek., 2,7 Sek. betragen.

Somit ist aus diesem Beispiel ersichtlich, dass die Atemfrequenz infolge der Anwendung des Verfahrens bei unterschiedlichen Funktionsaktivitäten geringer (oder das die Phasendauer des Atmungszyklus größer) wird, als bei natürlicher Atmung (wenn das Verfahren nicht benutzt wird). Dank einer solchen Phasenverlängerung des Atmungszyklus (die Verringerung der Atemfrequenzen) wird die vorgegebene dosierte Hypoxie bei unterschiedlichen Funktionsaktivitäten (bei verschiedenen Tätigkeiten) erzeugt.

Dabei wird auch der Bedarf an Sauerstoffversorgung des Organismus bei unterschiedlichen Funktionsaktivitäten (bei verschiedenen Tätigkeiten) objektiv berücksichtigt. Aus dem Beispiel ist ersichtlich, dass bei der Aktivitätserhöhung von 1 (mit HKF = 60 Schläge/Min) zu 2 (mit HKF = 90 Schläge/Min) und dann zu 3 (mit HKF = 110 Schläge/Min) die Erhöhung der Atemfrequenzen zur Versorgung der neuen Sauerstoffbedürfnisse des Organismus zustande kommt, wie es bei natürlicher Atmung der Fall ist.

In diesem Fall wird die Atemregelung folgenderweise realisiert: Der Benutzer gibt in der Mobileinrichtung den N-Wert für die Anzahl der Herzkontraktionen vor. Während dieser Zeit wird jede Phase des Atmungszyklus ausgeführt. Der Benutzer kann diese Einrichtung immer mittragen und jede Phase des Atmungszyklus nach dem Signal von der Einrichtung ausführen. Die Einrichtung zählt die Anzahl der Herzkontraktionen aus. Sobald der Zählwert dem vorgegebenen N-Wert gleich wird, gibt die Einrichtung dem Benutzer ein Signal aus. Dieses Signal bedeutet den Anfang der Ausführung der nächsten Phase des Atmungszyklus. Danach wird der Zähler in der Einrichtung rückgesetzt, und der Zählzyklus wiederholt sich. Dadurch wird die Atemregelung sichergestellt, solange das Verfahren benutzt wird. Der Wirkungsgrad (Sauerstoffmangel, -überschuss) beim Einsatz des Verfahrens wird von dem N-Wert abhängig sein. Ähnlich wie für das rhythmische Atmen, wird empfohlen, den N-Wert (Belastungsgrad) allmählich je nach Geübtheit sowie je nach dem Trainingsziel zu erhöhen. Gleich am Anfang der Anwendung des Verfahrens wird einige Zeit zur Gewöhnung an ein solches Verfahren der Atemregelung benötigt (um den bedingten Reflex auf das Steuersignal zu entwickeln). Ferner wird das Verfahren in der Form der bedingt-reflektorischen Atemregelung angewendet. Die Konzentration der Aufmerksamkeit auf die Atemregelung ist nicht mehr erforderlich. Dies ermöglicht, das Verfahren gleichzeitig mit anderen Tätigkeiten (Funktionsaktivitäten) anzuwenden (z. B. Haushaltsarbeiten, Fernsehen, Arbeit auf dem PC usw.). Somit ermöglicht die Atemregelung, die Einwirkungszeit auf den Körper (der Organismus befindet sich im Zustand des gewissen Sauerstoffmangels) zu verlängern. Damit wird die bestimmte dosierte Hypoxie erreicht und gleichzeitig mit anderen Tätigkeitsarten kombiniert. Je länger das Verfahren zur Atemregelung benutzt wird desto beständiger wird der Reflex, die Phasen des Atmungszyklus nach dem Signal einzuhalten, und umgekehrt. Je besser der Reflex erhalten bleibt, desto einfacher und bequemer wird der Vorgang der Atemregelung.

Um das Verfahren bei verschiedenen Tätigkeiten anzuwenden, muss die Einrichtung mobil, zierlich wie die meisten Mobileinrichtungen (Handy, Flash-Player, Taschenempfänger usw.) sein. Deswegen kann diese Einrichtung praktisch immer und bei verschiedenen Tätigkeiten (Funktionsaktivitäten) leicht und bequem benutzt werden. Bei der Anwendung des Verfahrens kann die Einrichtung neben dem Benutzer gelegt oder mitgetragen werden (genau so wie ein Handy, das sich in der Nähe des Benutzers befindet oder in der Tasche usw. des Benutzers getragen werden kann usw.).

Die modernen Sensoren ermöglichen es, die Funktionsparameter des Körpers kontaktlos zu erfassen. Es kann auch ein Geber nach dem Typ der Sport-Kardiomonitore (Brustkardiomonitore) sein. Von diesem Geber aus werden die Signale über einen Funkkanal in die Einrichtung übertragen. Diese Signale entsprechen den Herzkontraktionen. Der Benutzer kann dabei auch Kopfhörer anschließen. Die Lautstärke des Signals kann wie erwünscht eingestellt und sowohl das Steuersignal von der Einrichtung als auch alle Außengeräusche gehört werden, ohne andere Personen zu stören. Der Anwender kann sogar sich am Gespräch beteiligen, d. h., er kann während des Trainings ebenso sozialorientiert und aktiv bleiben, wie es ohne Benutzung dieses Verfahrens gemäß der Anmeldung der Fall ist. Von der Seite gesehen, wird ein Mensch während des Trainings kaum als solcher wahrgenommen: Er sieht aus wie ein Mensch mit Kopfhörer, der ganz normal atmet (d. h., ohne jegliche Zwischenelemente zwischen den Atmungsorganen und der Umwelt). Das ermöglicht auch, das vorgeschlagene Verfahren bei verschiedenen Tätigkeiten anzuwenden, weil die Umgebung nur die Köpfhörer und vielleicht die Einrichtung wahrnimmt. Dabei sieht die letztere öfters als ein Flash-Player bzw. als ein Taschenempfänger aus. Die eigentliche Atmung, ob beschleunigt oder selten, ist von der Frequenz her nur wenig bemerkbar.

Das Steuersignal von der Mobileinrichtung kann (bevorzugt) akustisch und/oder visuell oder anderweitig, zum Beispiel, ein Vibrationssignal sein. Am einfachsten wird das Verfahren für die Zweiphasenatmung bei zeitmäßig gleichem Ein- und Ausatmen, besonders am Anfang der Anwendung des Verfahrens benutzt. Es ist kompliziert, nach dem ersten Einatmen die Luft nochmals einzuatmen. Das Ausatmen nach dem Signal erfolgt automatisch, ohne Anstrengungen. Es ist auch bequem, am Anfang des Trainings sich nach dem Signal abzustimmen: Man muss mit jener Phase - Ein- oder Ausatmen - anfangen, welche bequemer ist. Um die Signale bei ungleichen Phasen voneinander zu unterscheiden (je nach der Übungsart), können die Signale von der Einrichtung, z. B., verschiedene Pegel aufweisen oder unterschiedlich frequenzmoduliert sein. D. h., für das Einatmen wird ein Typ der Signale (mit höherer Modulation) und für das Ausatmen - ein anderer Typ (niedrigere Modulation) verwendet. Oder das Einatmen kann mit dem Signal zu einem Kopfhörer und das Ausatmen mit dem Signal zu einem anderen Kopfhörer gekennzeichnet werden. Bei der Vierphasenatmung kann eine eigene Signalkombination benutzt werden. Oder es können die Meldungen über die jeweilige auszuführende Phase direkt auf einem Display der Einrichtung angezeigt werden. Es kann auch eine Kombination von akustischen Signalen benutzt werden, die zu verschiedenen Kopfhörern gerichtet werden und/oder in ihren Kennwerten unterschiedlich sein können.

Die gleichzeitige Verbindung des rhythmischen Atmens (um die Atemfrequenzen zu verändern) mit unterschiedlichen Tätigkeitsarten (Funktionsaktivitäten) ist ohne das vorgeschlagene Verfahren praktisch unmöglich. Das hängt damit zusammen, dass die eventuelle Tätigkeitsablösung oder ihre Intensität, die HKF-Änderung herbeiführt. Die HKF-Änderungen können innerhalb von einer kurzen Zeitspanne ziemlich beträchtlich sein. Ohne entsprechende technische Mittel ist der Mensch nicht imstande, den Atemrhythmus und den Herzrhythmus aufeinander abzustimmen. Das angewandte Verfahren erhält eine bestimmte Hypoxie bei der Änderung des Funktionszustandes sachlich, ohne menschlichen Eingriff. Die Sensorimpulse werden ständig durch die Einrichtung empfangen. Die Änderung der Herzkontraktionsfrequenz bei beliebigen Funktionsaktivitäten (Tätigkeit) verursacht Phasendauervariationen des Atmungszyklus gemäß dem empfangenen Signal. Das stellt die Aufrechterhaltung der Hypoxie im Körper während der Funktionszustandsänderung bei beliebigen Funktionsaktivitäten (Tätigkeit oder Ruhe) zum jetzigen Zeitpunkt sicher. Bei der Intensivierung (oder Reduzierung) der Funktionsaktivitäten und/oder des Einflusses anderer Außenfaktoren wird die HKF dementsprechend erhöht (vermindert). Folglich wird der Herzkontraktionszähler in der Einrichtung die N Herzkontraktionen innerhalb von einer kürzeren (oder jeweils längeren) Zeitspanne auszählen. Im Endeffekt wird die Dauer des Atmungszyklus verkürzt (oder dementsprechend verlängert). Der Sauerstoffversorgungsgrad des Organismus wird erhöht (vermindert), wobei die vorgegebene dosierte Hypoxiestufe erhalten bleibt.

Die Phasendauer des Atmungszyklus hängt von der Frequenz der Herzkontraktionen ab. Dabei wird der Einfluss von Außen- und Innenfaktoren auf den Sauerstoffbedarf des Organismus korrigiert. Es wird ermöglicht, das Verfahren bei einer beliebigen Tätigkeit zu benutzen, indem die Objektivität der Verfolgung der Sauerstoffbedarfsänderungen im Körper je nach dem Einfluss der sich abwechselnden Außen- und inneren Faktoren sichergestellt wird.

Somit wird der Übungsvorgang bei beliebigen Funktionsaktivitäten (Tätigkeit) realisiert. Es wird die Möglichkeit zur dauerhaften Anwendung des Verfahrens zur Erzeugung der dosierten Hypoxie gegeben. Es wird die bestimmte dosierte Hypoxie im Körper innerhalb einer langen Zeit aufrechterhalten. Beliebige Tätigkeit (funktionelle Aktivität, darunter auch der Ruhezustand als eine der Abarten der Funktionsaktivitäten), bei der das Verfahren zur Atemregelung benutzt wird, verlängert die Zeit der dosierten Hypoxie im Organismus.

Die modernen Technologien ermöglichen es, eine solche Einrichtung leicht und mobil auszuführen und sie mit komplizierten Funktionen (Algorithmen) der Atemregelung je nach der Herzkontraktionsfrequenz und anderen erfassbaren Funktionsparameter des Organismus (arterieller Druck, elektrische Hautleitfähigkeit, Sauerstoffmenge oder Kohlensäuremenge usw.) zu versehen. Als Einrichtungen zur Realisierung des betrachteten Verfahrens zur Atemregelung können zum Beispiel Taschen-PCs oder Mikrosteuerungen-Mikrochips (mit einem Prozessor, einem Speicher, Datenein- und -ausgabekanälen; sie werden je nach der darin gespeicherten SW in Plastikkarten, Handys, Uhren usw. benutzt) eingesetzt werden. Die Realisierung der Einrichtung zur momentanen Erfassung der Werte der körperlichen Funktionsparameter des Benutzers auf der Basis dieser elektronischen Geräte und zur Vorgabe des erforderlichen Atemrhythmus stellt eine technisch ausführbare Aufgabe dar. Im Speicher dieser elektronischen Geräte kann ein individuelles Programm (das Verhältnis der Phasendauer des Atmungszyklus zu HKF, Sauerstoffgehalt im Blut und Kohlendioxid usw.) für den optimalen Ablauf des Trainingsvorgangs (Atemregelung) aufgenommen werden. Dieses Programm kann unter Einsatz von Tests auf einem Fahrradtrainer mit der Erfassung von verschiedenen Funktionskennwerten (arterieller Druck, elektrische Hautleitfähigkeit, Sauerstoffmenge oder Kohlensäuremenge im Blut, Oxymetrie usw.) bei unterschiedlichen Belastungsarten und HKF erstellt werden. Nach der Aufnahme dieser Daten in die Einrichtung bekommt der Benutzer während des Trainings das Signal von der Einrichtung für die Ausführung der jeweiligen Atmungszyklusphasen. Die Folgefrequenz dieses äußeren Steuersignals entspricht dem aktuellen funktionellen Zustand des Organismus, dem Sauerstoffbedarf des Organismus und der vorgegebenen Trainingsbelastung.

Die modernen Mobileinrichtungen verfügen über eine umfangreiche Speicherkapazität. In diesen Speicher können verschiedene Trainingspläne (Algorithmen zur Erzeugung des Steuersignals je nach den funktionellen Parametern des Organismus) aufgenommen und später je nach dem Trainingsziel benutzt werden. Somit wird der Benutzer nur den erforderlichen Trainingsplan wählen und gemäß dem Signal von der Mobileinrichtung beim Training atmen müssen. Das Training kann gleichzeitig mit anderen Tätigkeitsarten erfolgen, so wie es zum Beispiel beim Musikhören gleichzeitig mit dem Autofahren der Fall ist.

## Patentansprüche

1. Verfahren zur rhythmischen Atmung,
**dadurch gekennzeichnet,**
**dass** die Phasendauer des Atmungszyklus nach einem Signal einer Mobileinrichtung bei unterschiedlichen Tätigkeiten (Funktionsaktivitäten) eingestellt wird, wobei die Phasendauer des Atmungszyklus innerhalb eines großen Zeitintervalls je nach dem Bestimmungszweck der Regelung geregelt wird.
